# EUROPÄISCHE PATENTSCHRIFT

(11) **EP 3 744 270 B1**
(45) Veröffentlichungstag und Bekanntmachung des Hinweises auf die Patenterteilung: **20.04.2022**
(21) Anmeldenummer: 20176170.7
(22) Anmeldetag: 22.05.2020
(51) Int. Cl.: A61B 17/34, A61B 90/50, A61B 17/17

(54) **SYSTEM ZUR POSITIONIERUNG**
SYSTEM FOR POSITIONING
SYSTÈME DE POSITIONNEMENT

(30) Priorität: 28.05.2019 DE 102019114352
(43) Veröffentlichungstag der Anmeldung: 02.12.2020
(73) Patentinhaber: Karl Storz SE & Co. KG Intellectual Property, 78532 Tuttlingen (DE)
(72) Erfinder: Kielack, Robin, 78532 Tuttlingen (DE); Nann, Steffen, 78532 Tuttlingen (DE); Sauer, Michael, 78532 Tuttlingen (DE)
(74) Vertreter: Witte, Weller & Partner Patentanwälte mbB

(56) Entgegenhaltungen:
- US-A- 6 071 288
- US-A1- 2003 051 591
- US-A1- 2004 220 588
- US-A1- 2008 027 457
- US-A1- 2009 163 766
- US-A1- 2011 313 478
- US-A1- 2017 258 659

## Beschreibung

Die vorliegende Erfindung betrifft ein System zur Positionierung, wobei das System insbesondere als Triangulationshilfe ausgebildet ist.

Bei einigen Operationen ist es schwierig, ein chirurgisches Instrument an einer definierten Stelle zu positionieren. Es stellt zudem eine zusätzliche Herausforderung dar, denselben Arbeitsbereich des chirurgischen Instruments mit einem Abbildungssystem zu visualisieren. Solche Schwierigkeiten ergeben sich beispielsweise bei Arbeiten innerhalb eines Gelenks, weil der Arbeitsbereich dann oftmals nur auf wenigen Wegen, insbesondere durch einen Gelenkspalt, erreicht werden kann. Dies soll nachfolgend am Bespiel einer Kiefergelenkarthroskopie verdeutlicht werden.

Der schwerwiegendste und zeitaufwendigste Schritt bei der Durchführung einer Kiefergelenkarthroskopie ist die sogenannte Triangulation, d.h. das Auffinden des Arbeitskanals mit dem Endoskop und das gemeinsame Navigieren von Arbeitskanal und Arthroskop durch den Gelenkspalt. Ziel ist es dabei, bestimmte chirurgische Arbeitsschritte unter endoskopischer Sicht im Gelenkspalt durchzuführen. Bei komplexen arthroskopischen Mikrooperationen ist eine detailgetreue Spiegelung der Innenstrukturen des Gelenks mit entsprechend hoher Bildqualität des Arthroskops nötig. Daher werden hier häufig KARL STORZ HOPKINS^{®}-Arthroskope benutzt.

Um Instrumente in das Gelenk einführen zu können, werden zusätzliche Trokare positioniert. Eine Reihe von Instrumenten, wie Fasszangen, Scheren, Tasthaken, Sichelmesser und Sonden, können über die Trokare im Gelenk angewendet werden. Die erfolgreiche Positionierung von Arthroskop und Trokaren basiert bisher ausschließlich auf der langjährigen Erfahrung des Anwenders und ist daher nur von Experten durchführbar.

US 2003/0051591 A1 offenbart eine Zielvorrichtung zur Positionierung eines Bohrwerkzeugs in Bezug auf einen zu erstellenden Bohrkanal in einem Knochen bestehend aus einem Zielhaken und einem mit diesem lösbar verbundenen Führungsarm, an dem eine Aufnahme für das Bohrwerkzeug angeordnet ist, und einer Führungsbahn in Form einer seitlich durchgehenden offenen Nut für den Führungsarm. Der Führungsarm ist in der Führungsbahn verschiebbar und fixierbar. Eine einfache Montage und Demontage der Vorrichtung wird dadurch erreicht, dass der Führungsarm an der Seite der Vorrichtung durch die Nutöffnung in die Führungsbahn eingebracht wird und umgekehrt durch Abheben aus der Nut vom Zielhaken getrennt werden kann.

US 2011/0313478 A1 offenbart eine chirurgische Führungsvorrichtung für die Rekonstruktion des vorderen Kreuzbandes, die einen Führungsarm und einen sich von dem Arm weg erstreckenden Führungshaken aufweist. Der Führungsarm ist bogenförmig ausgebildet, trägt eine Halterung, die auf dem Führungsarm verschiebbar ist, und hat eine von der Halterung getrennte Befestigungsvorrichtung für einen Ausrichtungsdraht.

US 2009/0163766 A1 offenbart eine Führungsbaugruppe, die eine Führung mit einem Körper und einem Gelenk mit mindestens einem Durchgangsloch, wobei das Gelenk zum Gleiten entlang einer Länge des Körpers konfiguriert ist, und eine erste chirurgische Vorrichtung umfasst. Die Führung ist mit der ersten chirurgischen Vorrichtung gekoppelt und eine Längsachse des Durchgangslochs ist koradial mit einem Ende der ersten chirurgischen Vorrichtung. Andere Führungsbaugruppen und Verfahren zur Erzeugung mehrerer Portale während der Operation sind ebenfalls offengelegt.

Es ist daher eine Aufgabe, ein verbessertes System zur Positionierung aufzuzeigen, mit dem insbesondere ein Abbildungssystem und ein chirurgisches Instrument auch an schwerzugänglichen Stellen zuverlässig und präzise positioniert werden können. Dabei soll insbesondere eine Kiefergelenkarthroskopie erleichtert werden.

Gemäß einem Aspekt wird diese Aufgabe gelöst durch ein System nach Anspruch 1.

Eine der Anwendungsmöglichkeiten dieses Systems ist wie folgt. Zunächst wird das erste Arbeitselement derart positioniert, dass der erste rohrförmige Schaft zumindest in der Nähe des gewünschten Arbeitsbereichs positioniert wird. Da der erste Schaft typischerweise ein Abbildungssystem aufweist, also einen Bildgeber führt oder Licht mittels eines optischen Systems zu einem Bildgeber leitet, kann die korrekte Positionierung des ersten Arbeitselements auch unmittelbar überprüft werden. Hierbei handelt es sich insbesondere um die Positionierung eines distalen Abschnitts eines Videoendoskops, entweder mit distalem Bildgeber oder einem proximal angeordneten Kamerakopf.

Anschließend wird das Bogenelement an dem ersten Arbeitselement fixiert. Konkret wird dabei ein Befestigungselement des Halteelements an dem Aufnahmeabschnitt des ersten Arbeitselements befestigt. Die Befestigung wird dabei insbesondere so gewählt, dass das Bogenelement starr am ersten Arbeitselement befestigt ist. Mit anderen Worten, es gibt kein bzw. nur sehr geringes relatives Spiel zwischen dem Bodenelement und dem ersten Arbeitselement.

Wenn zu diesem Zeitpunkt das Führungselement noch nicht auf der Schiene des Bogenelements angeordnet ist, wird das Führungselement nun auf der Schiene angeordnet. Dafür gibt es insbesondere die zwei folgenden Möglichkeiten. Zum einen kann das Führungselement mit dem Halteabschnitt entlang der Bogenrichtung auf die Schiene aufgesteckt werden. Dies kann, je nach Ausgestaltung, auch dann durchgeführt werden, wenn sich das Verriegelungselement bereits ganz oder teilweise in der Verriegelungsposition befindet. Zum anderen kann der Halteabschnitt seitlich auf die Schiene gesetzt werden, also aus der Querrichtung. In diesem Fall befindet sich das Verriegelungselement in der Freigabeposition. Wenn das Führungselement dann auf der Schiene angeordnet ist, wird das Verriegelungselement teilweise oder ganz in die Verriegelungsposition gebracht, um nun eine Verlagerung in Querrichtung zu blockieren. Es sei darauf hingewiesen, dass die Bogenrichtung, die Zentrumsrichtung und die Querrichtung ein Zylinderkoordinatensystem bilden.

Es kann nun das zweite Arbeitselement in den Führungsabschnitt des Führungselements eingeführt werden. Dabei wird das zweite Arbeitselement mittels des Führungsabschnitts senkrecht zur Bogenrichtung, also in Zentrumsrichtung, zum Arbeitsbereich geführt, der zu Beginn mittels des ersten Arbeitselements identifiziert und lokalisiert wurde. Es kann nun ein chirurgisches Element in den zweiten rohrförmigen Schaft des zweiten Arbeitselements eingeführt werden. Aufgrund der Ausrichtung des zweiten Arbeitselements in Richtung des Arbeitsbereichs, gelangt somit auch das chirurgische Element zum Arbeitsbereich. Da das Abbildungssystem, der im ersten rohrförmigen Schaft geführt ist, den Arbeitsbereich visualisiert, ist mittels des Abbildungssystems nun auch die Ankunft des chirurgischen Instruments am Arbeitsbereich zu sehen. Es sei darauf hingewiesen, dass das zweite Arbeitselement auch mit darin bereits eingesetztem chirurgischem Instrument in den Führungsabschnitt eingeführt werden kann.

Nachdem nun das erste Arbeitselement und das zweite Arbeitselement wie gewünscht positioniert sind, wird das Bogenelement nicht mehr benötigt. Daher wird nun das Verriegelungselement von der Verriegelungsposition in die Freigabeposition überführt. Auf diese Weise kann das Führungselement nun seitlich von der Schiene des Bogenelements abgezogen werden. Dabei entsteht nur eine geringe Verlagerung des zweiten Arbeitselements, wodurch das Trauma beim Patienten gering gehalten wird. Es ist zudem möglich, das erste Arbeitselement einschließlich des Bogenelements geringfügig um die erste Längsmittelachse zu drehen. Auf diese Weise bewegt sich die Schiene aus dem Halteabschnitt heraus, ohne dass das Führungselement verlagert werden muss. Auf diese Weise bleibt das zweite Arbeitselement in seiner Positionierung im Wesentlichen unverändert, so dass das Trauma für den Patienten beim Trennen von Führungselement und Bogenelement minimal ist. Zwar ist es grundsätzlich auch möglich, das Führungselement entlang der Bogenrichtung von der Schiene abzuziehen, doch wird das im Hinblick auf das möglicherweise größere Trauma als weniger bevorzugt angesehen.

Wenn das Führungselement vom Bogenelement gelöst ist, kann das Halteelement des Bogenelements vom Aufnahmeabschnitt des ersten Arbeitselements gelöst werden. Es kann nun ein freies Arbeiten unter Verwendung des ersten Arbeitselements und des zweiten Arbeitselements erfolgen. Es sei darauf hingewiesen, dass eine erneute Positionierung vorgenommen werden kann, indem das Halteelement erneut auf den Aufnahmeabschnitt gesetzt wird und das Führungselement erneut auf die Schiene gesetzt wird.

Somit wird unter anderem ein Zusammenführen des ersten Schafts und des zweiten Schafts in einem Gelenk ermöglicht, insbesondere in einem Kiefergelenk, ohne den gewünschten Navigationsbereich des Anwenders bezüglich des ersten Arbeitselements und des zweiten Arbeitselements im weiteren Verlauf des medizinischen Eingriffs zu beschränken. Es sei darauf hingewiesen, dass bei bestimmten Ausführungsformen der erste Schaft als Optikschaft und der zweite Schaft als Instrumentenschaft bezeichnet werden kann. Der Querschnitt der Schiene, also senkrecht zur Bogenrichtung, ist bevorzugt rechteckig mit gerundeten Ecken oder quadratisch mit gerundeten Ecken.

Das Befestigungselement weist einen Wipphebel auf, der an einem ersten Ende einen ersten Vorsprung aufweist und an einem gegenüberliegenden zweiten Ende eine Bedienfläche aufweist, wobei der Vorsprung dafür ausgebildet ist, in eine Nut des Aufnahmeabschnitts einzugreifen, und der Wipphebel dafür ausgebildet ist, dass die Befestigung am Aufnahmeabschnitt bei Betätigung des Wipphebels mit einer Hand gelöst oder hergestellt werden kann.

Dies bietet eine gute Möglichkeit, das Halteelement am Aufnahmeabschnitt zu befestigen. Dabei stellt der Vorsprung insbesondere die Negativform der Nut dar, so dass eine zumindest im Wesentlichen spielfreie Befestigung ermöglicht ist.

Somit ist die Aufgabe vollständig gelöst.

Bei einer vorteilhaften Ausgestaltung weist der Halteabschnitt ein Nut auf, die die Schiene aufnehmen kann, wobei das Verriegelungselement dafür ausgebildet ist, die offene Seite der Nut zu verengen oder zu verschließen und so die Verlagerung des Halteabschnitts in der Querrichtung zu blockieren.

Diese Ausgestaltung ist einfach und robust. Es kann auf diese Weise auch ein Verklemmen des Führungselements an der Schiene verhindert werden.

Bei einer weiteren vorteilhaften Ausgestaltung weist die Schiene eine Fase auf, die dem Verriegelungselement zugewandt ist.

Diese Ausgestaltung bietet eine konstruktiv einfache Möglichkeit, eine Verlagerung des Führungselements in Querrichtung zu blockieren.

Bei einer weiteren vorteilhaften Ausgestaltung ist das Verriegelungselement als Hebel ausgebildet, der von der Verriegelungsposition in die Freigabeposition und von der Freigabeposition in die Verriegelungsposition verschwenkt werden kann.

Diese Ausgestaltung ermöglicht eine einfache mechanische Betätigung. Dabei kann das Verriegelungselement insbesondere derart ausgestaltet sein, dass es in der Verriegelungsposition mit einer schrägen Fläche an der Fase anliegt. In der Freigabeposition entfernt sich der Hebel so weit von der Fase, dass die Schiene freigegeben wird.

Bei einer weiteren vorteilhaften Ausgestaltung kann das Verriegelungselement mittels einer Stellschraube von der Verriegelungsposition in die Freigabeposition und von der Freigabeposition in die Verriegelungsposition verlagert oder verschwenkt werden.

Diese Ausgestaltung ermöglicht eine gut dosierbare Betätigung des Verriegelungselements. Dabei lassen sich insbesondere eine erste und eine zweite Verriegelungsposition realisieren. In der ersten Verriegelungsposition blockiert das Verriegelungselement eine Verlagerung des Halteabschnitts in der Querrichtung, erlaubt aber eine Verlagerung des Führungselements entlang der Schiene, also entlang der Bogenrichtung. Wird die Stellschraube weiter betätigt, drückt das Verriegelungselement schließlich gegen die Schiene, so dass sich eine zweite Verriegelungsposition einstellt, in der zusätzlich eine Verlagerung des Führungselements entlang der Schiene, also in Bogenrichtung, unterbunden wird. Mit anderen Worten, ist möglich, das Führungselement relativ zur Schiene zu fixieren.

Bei einer weiteren vorteilhaften Ausgestaltung weist der Aufnahmeabschnitt eine erste Nut auf, die zumindest im Wesentlichen parallel zur ersten Längsmittelachse verläuft.

Bei dieser Ausgestaltung bietet die Nut eine mechanisch zuverlässige Möglichkeit, das Befestigungselement des Halteelements auf dem Aufnahmeabschnitt zu befestigen. Bei dieser Ausgestaltung kann der Grundkörper des Aufnahmeabschnitts insbesondere ein Quader mit einem quadratischen Querschnitt bezogen auf die erste Längsmittelachse sein.

Bei einer weiteren vorteilhaften Ausgestaltung weist der Aufnahmeabschnitt eine zweite Nut auf, die zumindest im Wesentlichen parallel zur ersten Längsmittelachse verläuft und insbesondere, bezogen auf die erste Längsmittelachse, der ersten Nut gegenüber liegt.

Diese Ausgestaltung bietet einerseits die Möglichkeit, das Halteelement in verschiedenen Positionen am Aufnahmeabschnitt zu befestigen. Ferner können die erste und die zweite Nut für eine stabile Positionierung des Halteelements am Aufnahmeabschnitt verwendet werden.

Bei einer weiteren vorteilhaften Ausgestaltung weist der Aufnahmeabschnitt weitere Nuten auf, die zumindest im Wesentlichen parallel zur ersten Längsmittelachse verlaufen und mehrere Aufnahmemöglichkeiten bezogen auf einen Winkel um die erste Längsmittelachse ermöglichen.

Auch diese Ausgestaltung ermöglicht es, das Halteelement in verschiedenen Positionen am Aufnahmeabschnitt zu befestigen. Dabei ist es vorteilhaft, wenn die Nuten, bezogen auf die erste Längsmittelachse, um jeweils 90° voneinander beabstandet sind.

Bei einer weiteren vorteilhaften Ausgestaltung ist die Schiene derart gebogen, dass sich die Zentrumsrichtung und eine Verlängerung der ersten Längsmittelachse schneiden, wenn das Bogenelement mittels des Befestigungselements auf dem Aufnahmeabschnitt befestigt ist.

Diese Ausgestaltung hilft dem Benutzer, das zweite Arbeitselement so zu positionieren, dass das im zweiten Schaft geführte chirurgische Instrument mittels eines Abbildungssystems am oder im ersten Schaft visualisiert werden kann.

Bei einer weiteren vorteilhaften Ausgestaltung ist ein erstes Ende des ersten rohrförmigen Schafts und ein zweites Ende des zweiten rohrförmigen Schafts weniger als 15 mm, bevorzugt weniger als 10 mm und besonders bevorzugt weniger als 5 mm voneinander entfernt, wenn das Bogenelement mittels des Befestigungselements auf dem Aufnahmeabschnitt befestigt ist und das zweite Arbeitselement vollständig in den Führungsabschnitt eingeschoben ist.

Diese Ausgestaltung ermöglicht es, dass sowohl ein im ersten Schaft geführtes Abbildungssystem als auch ein im zweiten Schaft geführtes chirurgisches Instrument denselben Arbeitsbereich zuverlässig erreichen.

Es ist ferner vorteilhaft, wenn eine Verlängerung der zweiten Längsmittelachse eine Verlängerung der ersten Längsmittelachse schneidet, wenn das Bogenelement mittels des Befestigungselements auf dem Aufnahmeabschnitt befestigt ist. Außerdem ist es vorteilhaft, wenn der erste rohrförmige Schaft für die Aufnahme eines Abbildungssystems ausgebildet ist oder ein Abbildungssystem aufweist und der zweite rohrförmige Schaft für die Aufnahme eines chirurgischen Instruments ausgebildet ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in den Zeichnungen dargestellt und wird in der nachfolgenden Beschreibung näher erläutert. Es zeigen:
- Fig. 1: eine perspektivische Ansicht eines ersten Arbeitselements gemäß einer bevorzugten Ausführungsform;
- Fig. 2: eine perspektivische Ansicht eines zweiten Arbeitselements gemäß der bevorzugten Ausführungsform;
- Fig. 3: eine perspektivische Ansicht eines Bogenelements mit einem aufgesetzten Führungselement gemäß der bevorzugten Ausführungsform;
- Fig. 4: eine perspektivische Ansicht des Führungselements aus Fig. 3;
- Fig. 5: eine Schnittdarstellung durch das Bogenelement und das Führungselement aus Fig. 3;
- Fig. 6: eine seitliche Ansicht eines Systems zur Positionierung gemäß der bevorzugten Ausführungsform;
- Fig. 7: eine erste perspektivische Darstellung des Systems aus Fig. 6; und
- Fig. 8: eine zweite perspektivische Darstellung des Systems aus Fig. 6.

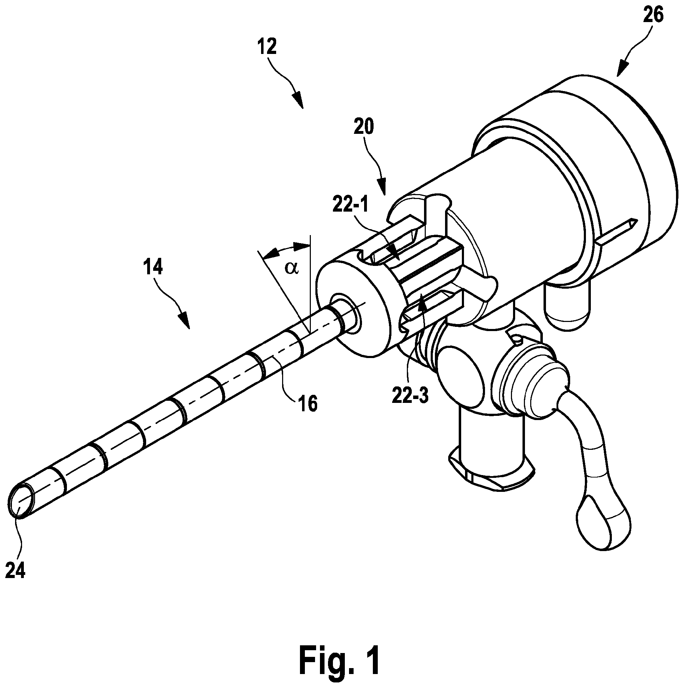

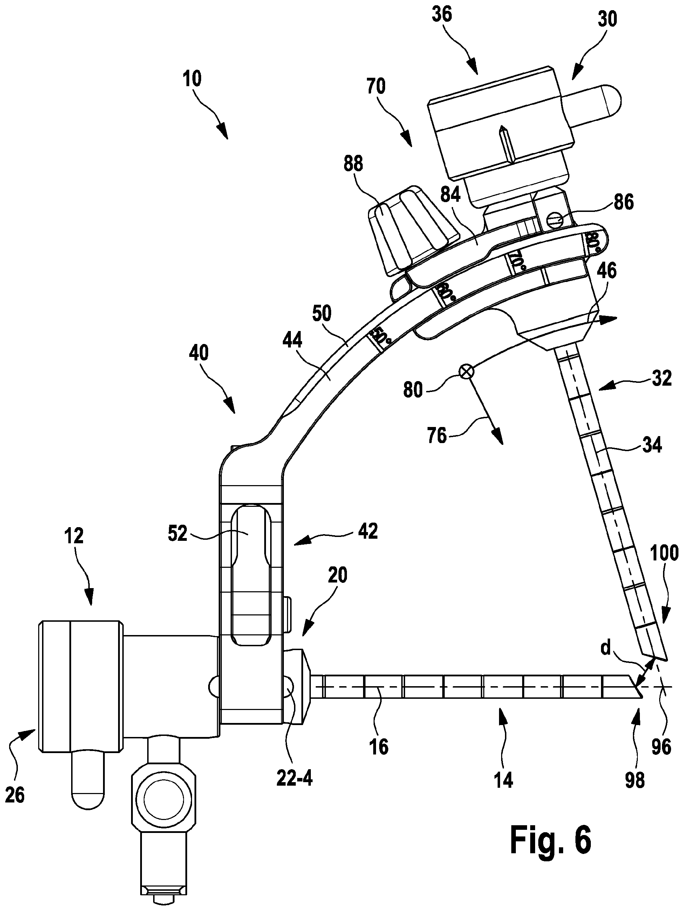

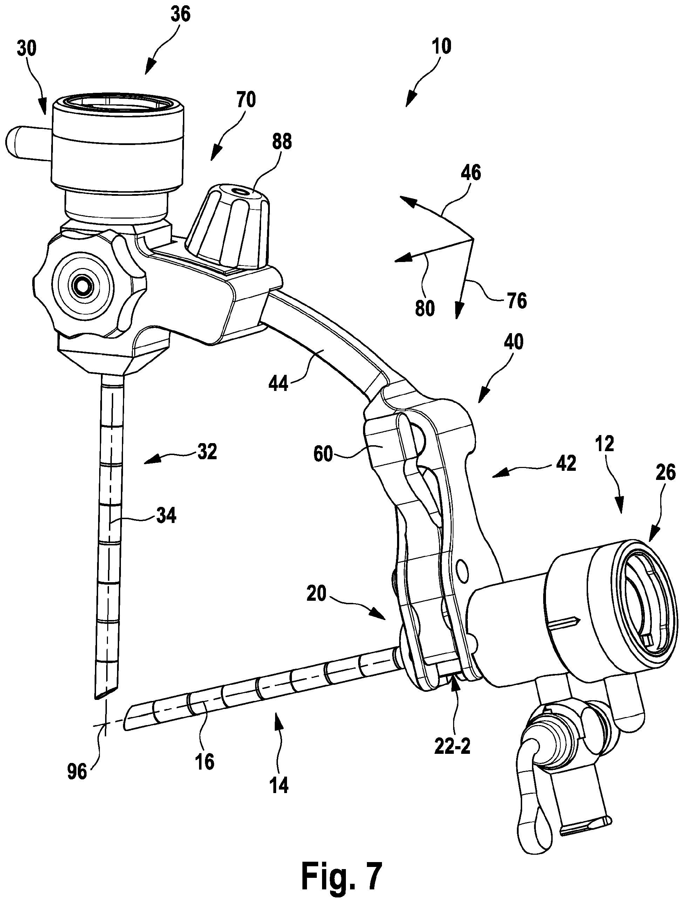

Fig. 1 zeigt ein erstes Arbeitselement 12 gemäß einer bevorzugten Ausführungsform. Das erste Arbeitselement 12 hat einen ersten rohrförmigen Schaft 14, der eine erste Längsmittelachse 16 aufweist. Ferner weist das erste Arbeitselement 12 einen Aufnahmeabschnitt 20 auf. Der Aufnahmeabschnitt 20 weist eine erste Nut 22-1 auf, die parallel zur ersten Längsmittelachse 16 erläuft. Der Aufnahmeabschnitt 20 weist ferner eine zweite Nut 22-2 (siehe Fig. 7) auf, die ebenfalls parallel zur ersten Längsmittelachse 16 verläuft und hier, bezogen auf die erste Längsmittelachse 16, der ersten Nut 22-1 gegenüberliegt. Der Aufnahmeabschnitt 20 weist zudem weitere Nuten auf, von denen in dieser perspektivischen Darstellung nur die weitere Nut 22-3 zu erkennen ist und die Nut 22-4 (siehe Fig. 6) verdeckt ist. Auch die weiteren Nuten verlaufen parallel zur ersten Längsmittelachse 16. Sie ermöglichen mehrere Aufnahmemöglichkeiten bezogen auf einen Winkel **α** um die erste Längsmittelachse 16.

Der Grundkörper des Aufnahmeabschnitts 20 ist hier ein Quader, dessen Querschnitt senkrecht zur ersten Längsmittelachse 16 quadratisch ist. In diesen Quader sind die Nuten 22-1, 22-2, 22-3 und 22-4 eingebracht.

Der erste Schaft 14 ist bei dieser Ausgestaltung mit einem optischen System 24 ausgefüllt. Licht, das vom Arbeitsbereich am distalen Ende des optischen Systems 24 einfällt, wird in Richtung der proximalen Seite des ersten Arbeitselements 12 geleitet und kann dort mittels eines Kamerakopfs (nicht gezeigt), der an einem ersten Anschluss 26 angeschlossen ist, erfasst werden.

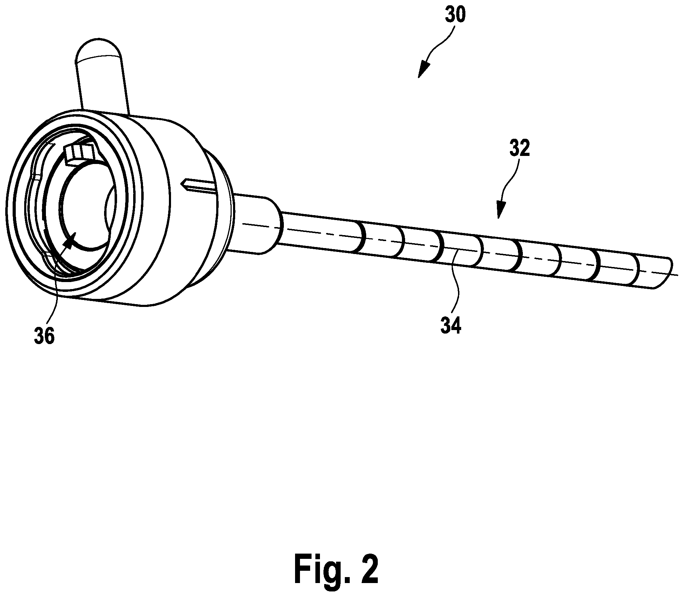

Fig. 2 zeigt ein zweites Arbeitselement 30 gemäß der bevorzugten Ausführungsform. Das zweite Arbeitselement 30 weist einen zweiten rohrförmigen Schaft 32 auf, der eine zweite Längsmittelachse 34 aufweist. Der zweite Schaft 32 ist für die Aufnahme eines chirurgischen Instruments (nicht gezeigt) ausgebildet. Das chirurgische Instrument kann über einen zweiten Anschluss 36 in den zweiten Schaft 32 eingeführt werden und an der Anschlussstelle auch verriegelt werden.

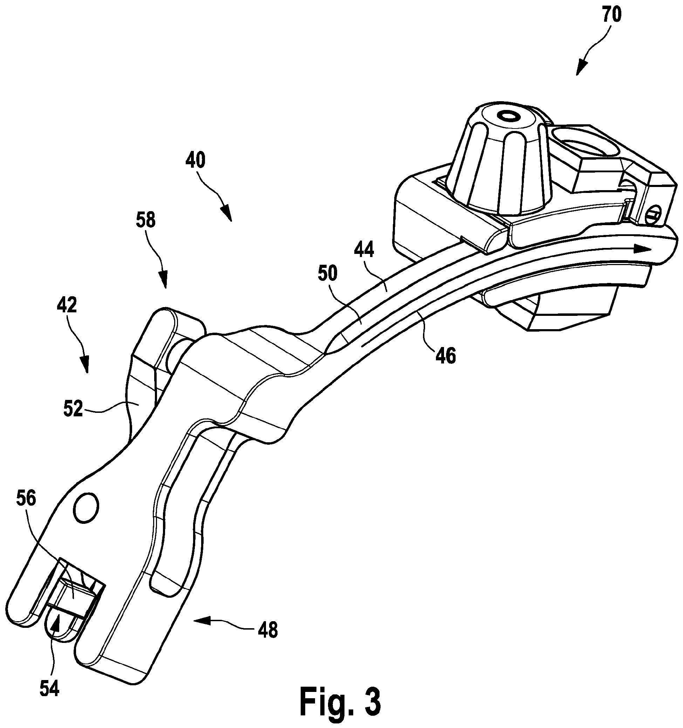

Fig. 3 zeigt ein Bogenelement 40 gemäß der bevorzugten Ausführungsform. Das hier ebenfalls gezeigte Führungselement 70 gemäß der bevorzugten Ausführungsform wird später erläutert.

Das Bogenelement 40 weist ein Halteelement 42 und eine bogenförmige Schiene 44 auf, die sich entlang einer Bogenrichtung, siehe Pfeil 46, erstreckt. Die Schiene 44 ist dabei an dem Halteelement 42 angeordnet.

Das Halteelement 42 ist dafür ausgebildet, auf dem Aufnahmeabschnitt 20 mittels eines Befestigungselements 48 lösbar und formschlüssig befestigt zu werden. Die Schiene 44 weist eine Fase 50 auf.

Das Befestigungselement 48 weist einen Wipphebel 52 auf, der an einem ersten Ende 54 einen ersten Vorsprung 56 aufweist und an einem gegenüberliegenden zweiten Ende 58 eine Bedienfläche 60 (siehe Fig. 7) aufweist. Der Vorsprung 56 ist dafür ausgebildet, in eine der Nuten 22-1, 22-2, 22-3, 22-4 des Aufnahmeabschnitts 20 einzugreifen. Es ist zu erkennen, dass das Befestigungselement 48 einen im Wesentlichen rechteckigen bzw. quadratischen Durchgang hat. Dieser Durchgang kann formschlüssig auf den Aufnahmeabschnitt 20 gesetzt werden, so dass bei der Befestigung nur ein geringes Spiel verbleibt. Wenn der erste Vorsprung 56 dann in eine der Nuten 22-1, 22-2, 22-3, 22-4 eingreift, ist das Bogenelement 40 lösbar am Aufnahmeabschnitt 20 befestigt.

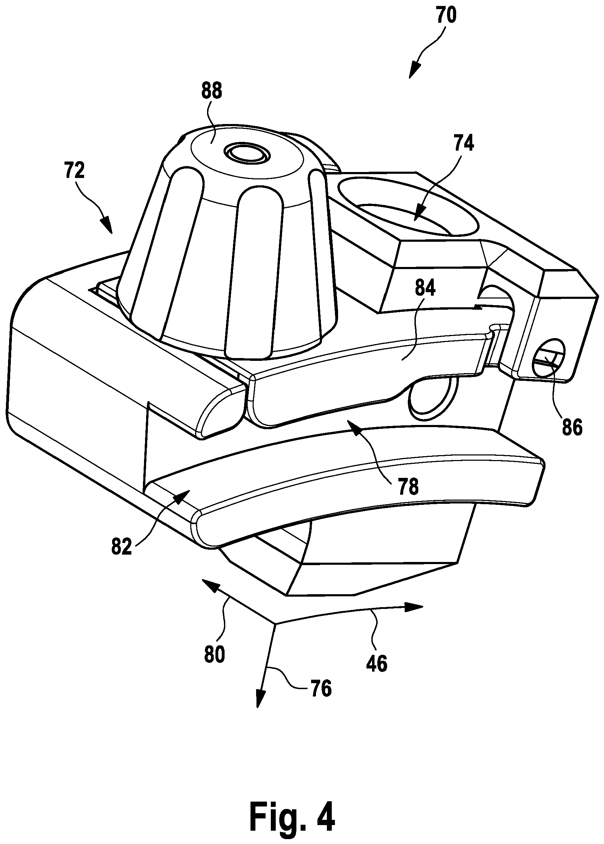

Fig. 4 zeigt das Führungselement 70 gemäß der bevorzugten Ausführungsform. Das Führungselement 70 hat einen Halteabschnitt 72 und einen Führungsabschnitt 74. Der Halteabschnitt 72 ist dafür ausgebildet, auf der Schiene 44 geführt zu werden. Der Führungsabschnitt 74 ist dafür ausgebildet, den zweiten rohrförmigen Schaft 32 aufzunehmen und in einer Zentrumsrichtung, Pfeil 76, zu führen, wenn der Halteabschnitt 72 auf der Schiene 44 geführt ist. Die Zentrumsrichtung 76 ist senkrecht zur Bogenrichtung 46. Der Halteabschnitt 72 weist ein Verriegelungselement 78 auf, das dafür ausgebildet ist, das Führungselement 70 an der Schiene 44 formschlüssig zu befestigen. Dabei ist das Verriegelungselement 78 dafür ausgebildet, in einer Verriegelungsposition eine Verlagerung des Halteabschnitts 72 in einer Querrichtung, in die Zeichenebene weisendes Richtungssymbol 80, zu blockieren. Die Querrichtung 80 ist dabei sowohl senkrecht zur Bogenrichtung 46 als auch Zentrumsrichtung 76. Das Verriegelungselement 78 ist ferner dazu ausgebildet, in einer Freigabeposition eine Verlagerung des Halteabschnitts 72 in der Querrichtung 80 zu ermöglichen.

Der Halteabschnitt 72 weist eine Haltenut 82 auf, die die Schiene 44 aufnehmen kann. Das Verriegelungselement 78 ist dafür ausgebildet, die offene Seite der Haltenut 82 zu verengen oder zu verschießen und so die Verlagerung des Halteabschnitts 72 in der Querrichtung 80 zu blockieren.

Das Verriegelungselement 78 ist als Hebel 84 ausgebildet, der von der Verriegelungsposition in die Freigabeposition und von der Freigabeposition in die Verriegelungsposition verlagert oder verschwenkt werden kann. Dies erfolgt hier jeweils um die Achse 86. Das Verriegelungselement 78 kann mittels einer Stellschraube 88 von der Verriegelungsposition in die Freigabeposition und von der Freigabeposition in die Verriegelungsposition verlagert oder verschwenkt werden. Optional kann eine Ausgestaltung gewählt werden, dass das Verriegelungselement 78 zudem gegen die Schiene 44 gedrückt werden kann, um so auch eine Verlagerung des Führungselements 70 entlang der Schiene 44 zu unterbinden.

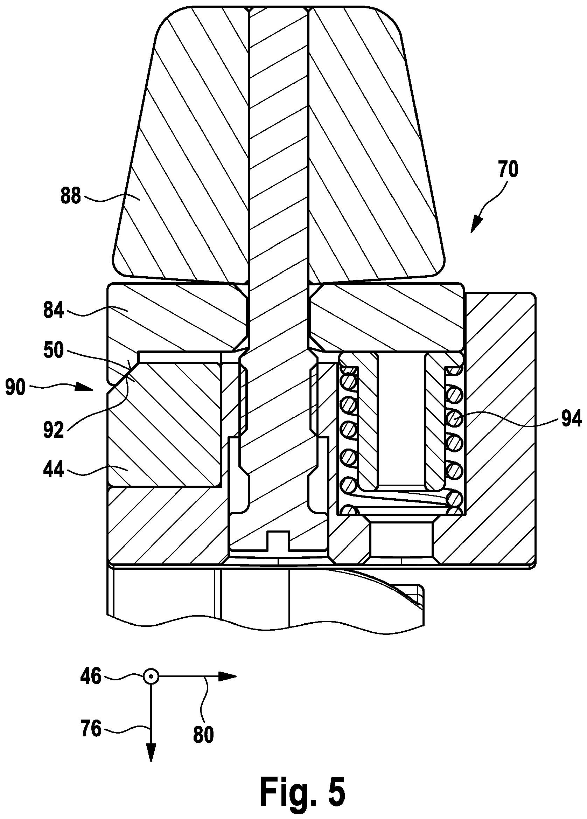

Fig. 5 zeigt wie bei der bevorzugten Ausführungsform das Zusammenspiel zwischen dem Hebel 84 des Verriegelungselement 78 und der Schiene 44 funktioniert. Es ist zu erkennen, dass ein Abschnitt 90 des Hebels 84 mit einer schrägen Fläche 92 an der Fase 50 der Schiene 44 anliegt. Das Führungselement 70 kann sich nun nicht in der Querrichtung 80 von der Schiene 44 lösen kann. Es ist ferner optional möglich, auf den Hebel 84 soviel Druck auszuüben, dass das Zusammenwirken der Fase 50 mit der schrägen Fläche 92 eine Verlagerung entlang der Bogenrichtung 46 verhindert. Wird die Stellschraube 88 gelockert, so drückt die Feder 94 den Hebel 84 nach außen, so dass sich die schräge Fläche 92 von der Fase 50 löst. Wenn der Hebel 84 sich weit genug nach außen verschwenkt hat, ist die Schiene 44 frei und das Führungselement 70 kann von der Schiene 44 gelöst werden.

Fig. 6 zeigt eine Seitenansicht eines Systems 10 zur Positionierung gemäß der bevorzugten Ausführungsform. Es ist zu erkennen, dass das Bogenelement 40 an dem Aufnahmeabschnitt 20 fixiert ist, dass das Führungselement 70 auf die Schiene 44 aufgesetzt ist und das zweite Arbeitselement 30 in den Führungsabschnitt 74 vollständig eingeschoben ist.

In dieser Fig. 6 ist ferner ein Schnittpunkt 96 gezeigt, an dem sich eine Verlängerung der ersten Längsmittelachse 16 und eine Verlängerung der zweiten Längsmittelachse 34 schneiden. Außerdem ist ein Abstand d eingezeichnet, der zwischen einem ersten Ende 98 des ersten rohrförmigen Schafts 14 und einem zweiten Ende 100 des zweiten rohrförmigen Schafts 32 besteht. Dieser Abstand d kann für eine genaue Positionierung sehr klein gewählt werden, besonders bevorzugt weniger als 5 mm.

Fig. 7 zeigt eine erste perspektivische Darstellung des Systems 10 aus Fig. 6.

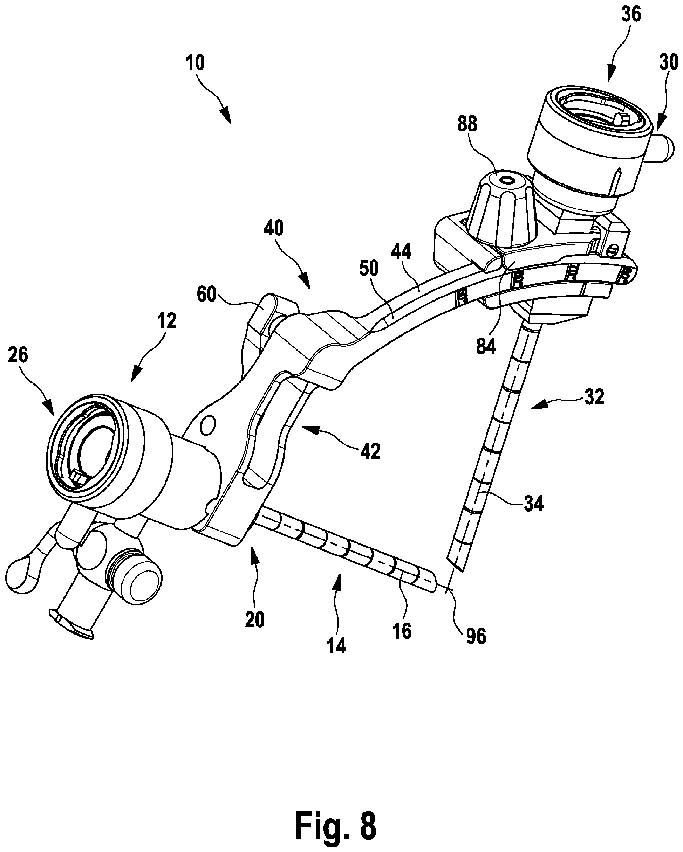

Fig. 8 zeigt eine zweite perspektivische Darstellung des Systems 10 aus Fig. 6.

## Patentansprüche

1. System (10) zur Positionierung aufweisend
ein erstes Arbeitselement (12) mit einem ersten rohrförmigen Schaft (14), der eine erste Längsmittelachse (16) aufweist, und mit einem Aufnahmeabschnitt (20),
ein zweites Arbeitselement (30) mit einem zweiten rohrförmigen Schaft (32), der eine zweite Längsmittelachse (34) aufweist,
ein Bogenelement (40) mit einem Halteelement (42) und einer bogenförmigen Schiene (44), die sich gebogen entlang einer Bogenrichtung (46) erstreckt, wobei die bogenförmige Schiene (44) an dem Halteelement (42) angeordnet ist und das Halteelement (42) dafür ausgebildet ist, auf dem Aufnahmeabschnitt (20) mittels eines Befestigungselements (48) lösbar und formschlüssig befestigt zu werden, und
ein Führungselement (70) mit einem Halteabschnitt (72) und einem Führungsabschnitt (74), wobei der Halteabschnitt (72) dafür ausgebildet ist auf der Schiene (44) geführt zu werden und der Führungsabschnitt (74) dafür ausgebildet ist den zweiten rohrförmigen Schaft (32) aufzunehmen und in einer Zentrumsrichtung (76), die senkrecht zur Bogenrichtung (46) ist, zu führen, wenn der Halteabschnitt (72) auf der Schiene (44) geführt ist, **dadurch gekennzeichnet dass**,
der Halteabschnitt (72) ein Verriegelungselement (78) aufweist, das dafür ausgebildet ist, das Führungselement (70) an der Schiene (44) formschlüssig zu befestigen, wobei das Verriegelungselement (78) in einer Verriegelungsposition eine Verlagerung des Halteabschnitts (72) in einer Querrichtung (80) blockiert und in einer Freigabeposition eine Verlagerung des Halteabschnitts (72) in der Querrichtung (80) ermöglicht, wobei die Querrichtung (80) senkrecht zur Bogenrichtung und senkrecht zur Zentrumsrichtung ist, und
wobei das Befestigungselement (48) einen Wipphebel (52) aufweist, der an einem ersten Ende (54) einen ersten Vorsprung (56) aufweist und an einem gegenüberliegenden zweiten Ende (58) eine Bedienfläche (60) aufweist, wobei der Vorsprung (56) dafür ausgebildet ist in eine Nut (22-1) des Aufnahmeabschnitts (20) einzugreifen und der Wipphebel (52) dafür ausgebildet ist, dass die Befestigung am Aufnahmeabschnitt (20) bei Betätigung des Wipphebels (52) mit einer Hand gelöst oder hergestellt werden kann.

2. System nach Anspruch 1, wobei der Halteabschnitt (72) eine Haltenut (82) aufweist, die die Schiene (44) aufnehmen kann, wobei das Verriegelungselement (78) dafür ausgebildet ist, die offene Seite der Haltenut (82) zu verengen oder zu verschließen und so die Verlagerung des Halteabschnitts (72) in der Querrichtung (80) zu blockieren.

3. System nach einem der vorhergehenden Ansprüche, wobei die Schiene (44) eine Fase (50) aufweist, die dem Verriegelungselement (78) zugewandt ist.

4. System nach Anspruch 3, wobei das Verriegelungselement (78) als Hebel (84) ausgebildet ist, der von der Verriegelungsposition in die Freigabeposition und von der Freigabeposition in die Verriegelungsposition verschwenkt werden kann.

5. System nach einem der vorhergehenden Ansprüche, wobei das Verriegelungselement (78) mittels einer Stellschraube (88) von der Verriegelungsposition in die Freigabeposition und von der Freigabeposition in die Verriegelungsposition verlagert oder verschwenkt werden kann.

6. System nach einem der vorhergehenden Ansprüche, wobei der Aufnahmeabschnitt (20) eine erste Nut (22-1) aufweist, die zumindest im Wesentlichen parallel zur ersten Längsmittelachse (16) verläuft.

7. System nach Anspruch 6, wobei der Aufnahmeabschnitt (20) eine zweite Nut (22-2) aufweist, die zumindest im Wesentlichen parallel zur ersten Längsmittelachse (16) verläuft und insbesondere, bezogen auf die erste Längsmittelachse (16), der ersten Nut (22-1) gegenüberliegt.

8. System nach Anspruch 6 oder 7, wobei der Aufnahmeabschnitt (20) weitere Nuten (22-3) aufweist, die zumindest im Wesentlichen parallel zur ersten Längsmittelachse (16) verlaufen und mehrere Aufnahmemöglichkeiten bezogen auf einen Winkel (α) um die erste Längsmittelachse (16) ermöglichen.

9. System nach einem der vorhergehenden Ansprüche, wobei die Schiene (44) derart gebogen ist, dass sich die Zentrumsrichtung (76) und eine Verlängerung der ersten Längsmittelachse (16) schneiden, wenn das Bogenelement (40) mittels des Befestigungselements (48) auf dem Aufnahmeabschnitt (20) befestigt ist.

10. System nach einem der vorhergehenden Ansprüche, wobei ein erstes Ende (98) des ersten rohrförmigen Schafts (14) und ein zweites (100) Ende des zweiten rohrförmigen Schafts (32) weniger als 15 mm, bevorzugt weniger als 10 mm und besonders bevorzugt weniger als 5 mm voneinander entfernt sind, wenn das Bogenelement (40) mittels des Befestigungselements (48) auf dem Aufnahmeabschnitt (20) befestigt ist und das zweite Arbeitselement (30) vollständig in den Führungsabschnitt (74) eingeschoben ist.

## Claims

1. A positioning system (10) comprising
a first working element (12) having a first tubular shaft (14) with a first longitudinal center axis (16) and having a receiving section (20),
a second working element (30) having a second tubular shaft (32) with a second longitudinal center axis (34),
an arc element (40) with a holding element (42) and a curved rail (44) extending curvedly along an arc direction (46), wherein the curved rail (44) is arranged on the holding element (42) and the holding element (42) is configured to be detachably and positively fastened on the receiving section (20) by a fastening element (48), and
a guiding element (70) having a holding section (72) and a guiding section (74), wherein the holding section (72) is adapted to be guided on the rail (44) and the guiding section (74) is adapted to receive the second tubular shaft (32) and guide it in a center direction (76) perpendicular to the arc direction (46) when the holding section (72) is guided on the rail (44),
**characterized in that** the holding section (72) has a locking element (78) which is configured for positively securing the guiding element (70) to the rail (44), wherein the locking element (78) blocks displacement of the holding section (72) in a transverse direction (80) in a locking position and allows displacement of the holding section (72) in the transverse direction (80) in a release position, wherein the transverse direction (80) is perpendicular to the direction of the arc and perpendicular to the center direction, and
wherein the fastening element (48) comprises a rocker lever (52) having a first projection (56) at a first end (54) and an actuation surface (60) at an opposite second end (58), wherein the projection (56) is adapted to engage in a groove (22-1) of the receiving section (20), and wherein the rocker lever (52) is configured such that the fastening at the receiving section (20), using the rocker lever (52) can be released or engaged with one hand.

2. The system according to claim 1, wherein the retaining section (72) has a retaining groove (82) which can receive the rail (44), wherein the locking element (78) is configured to narrow or close the open side of the retaining groove (82) and thus block the displacement of the retaining section (72) in the transverse direction (80).

3. The system according to one of the preceding claims, wherein the rail (44) has a chamfer (50) facing the locking element (78).

4. The system according to claim 3, wherein the locking element (78) is configured as a lever (84) which can be pivoted from the locking position into the release position and from the release position into the locking position.

5. The system according to one of the preceding claims, wherein the locking element (78) can be displaced or pivoted from the locking position to the release position and from the release position to the locking position by a set screw (88).

6. The system according to one of the preceding claims, wherein the receiving section (20) has a first groove (22-1) which is at least substantially parallel to the first longitudinal center axis (16).

7. The system according to claim 6, wherein the receiving section (20) has a second groove (22-2) which extends at least substantially parallel to the first longitudinal center axis (16) and, in particular, is opposite the first groove (22-1) with respect to the first longitudinal center axis (16).

8. The system according to claim 6 or 7, wherein the receiving section (20) has further grooves (22-3) which extend at least substantially parallel to the first longitudinal center axis (16) and permit a plurality of receiving facilities with respect to an angle (α) about the first longitudinal center axis (16).

9. The system according to one of the preceding claims, wherein the rail (44) is bent in such a way that the center direction (76) and an extension of the first longitudinal center axis (16) intersect when the arc element (40) is fixed on the receiving section (20) by the fastening element (48).

10. The system according to one of the preceding claims, wherein a first end (98) of the first tubular shaft (14) and a second (100) end of the second tubular shaft (32) are less than 15 mm, preferably less than 10 mm and more preferably less than 5 mm apart when the arc element (40) is fixed on the receiving section (20) by the fastening element (48) and the second working element (30) is fully inserted into the guiding section (74).

## Revendications

1. Système (10) de positionnement présentant
un premier élément de travail (12) doté d'une première tige tubulaire (14), qui présente un premier axe médian longitudinal (16), et d'une partie de réception (20),
un deuxième élément de travail (30) doté d'une deuxième tige tubulaire (32), qui présente un deuxième axe médian longitudinal (34),
un élément arqué (40) doté d'une partie de retenue (42) et d'un rail (44) en forme d'arc qui s'étend de manière arquée le long d'une direction d'arc (46), le rail (44) en forme d'arc étant disposé sur l'élément de retenue (42) et l'élément de retenue (42) étant réalisé pour être fixé de manière amovible et par complémentarité de formes sur la partie de réception (20) au moyen d'un élément de fixation (48), et
un élément de guidage (70) doté d'une partie de retenue (72) et d'une partie de guidage (74), la partie de retenue (72) étant réalisée pour être guidée sur le rail (44) et la partie de guidage (74) étant réalisée pour recevoir la deuxième tige tubulaire (32) et pour la guider dans une direction centrale (76), qui est perpendiculaire à la direction d'arc (46), lorsque la partie de retenue (72) est guidée sur le rail (44), **caractérisé en ce que** la partie de retenue (72) présente un élément de verrouillage (78) qui est réalisé pour fixer par complémentarité de formes l'élément de guidage (70) sur le rail (44), l'élément de verrouillage (78) bloquant, dans une position de verrouillage, un déplacement de la partie de retenue (72) dans une direction transversale (80) et permettant, dans une position de libération, un déplacement de la partie de retenue (72) dans la direction transversale (80), la direction transversale (80) étant perpendiculaire à la direction d'arc et perpendiculaire à la direction centrale, et
l'élément de fixation (48) présentant un levier basculant (52) qui présente une première saillie (56) à une première extrémité (54) et une surface de commande (60) à une deuxième extrémité (58) opposée, la saillie (56) étant réalisée pour venir en prise dans une rainure (22-1) de la partie de réception (20) et le levier basculant (52) étant réalisé pour que la fixation sur la partie de réception (20) puisse être relâchée ou produite par une main lors de l'actionnement du levier basculant (52).

2. Système selon la revendication 1, la partie de retenue (72) présentant une rainure de retenue (82) qui peut recevoir le rail (44), l'élément de verrouillage (78) étant réalisé pour rétrécir ou pour fermer le côté ouvert de la rainure de retenue (82) et pour ainsi bloquer le déplacement de la partie de retenue (72) dans la direction transversale (80).

3. Système selon l'une des revendications précédentes, le rail (44) présentant un biseau (50) qui est tourné vers l'élément de verrouillage (78).

4. Système selon la revendication 3, l'élément de verrouillage (78) étant réalisé sous la forme d'un levier (84) qui peut être pivoté de la position de verrouillage dans la position de libération et de la position de libération dans la position de verrouillage.

5. Système selon l'une des revendications précédentes, l'élément de verrouillage (78) pouvant être, au moyen d'une vis de réglage (88), déplacé ou pivoté de la position de verrouillage dans la position de libération et de la position de libération dans la position de verrouillage.

6. Système selon l'une des revendications précédentes, la partie de retenue (20) présentant une première rainure (22-1) qui s'étend au moins sensiblement parallèlement au premier axe médian longitudinal (16).

7. Système selon la revendication 6, la partie de retenue (20) présentant une deuxième rainure (22-2) qui s'étend au moins sensiblement parallèlement au premier axe médian longitudinal (16) et est en particulier, par rapport au premier axe médian longitudinal (16), opposée à la première rainure (22-1).

8. Système selon la revendication 6 ou 7, la partie de retenue (20) présentant d'autres rainures (22-3) qui s'étendent au moins sensiblement parallèlement au premier axe médian longitudinal (16) et permettent plusieurs possibilités de réception par rapport à un angle (a) autour du premier axe médian longitudinal (16).

9. Système selon l'une des revendications précédentes, le rail (44) étant arqué de telle sorte que la direction centrale (76) et un prolongement du premier axe médian longitudinal (16) se coupent lorsque l'élément arqué (40) est fixé sur la partie de réception (20) au moyen de l'élément de fixation (48).

10. Système selon l'une des revendications précédentes, une première extrémité (98) de la première tige tubulaire (14) et une deuxième extrémité (100) de la deuxième tige tubulaire (32) étant éloignées l'une de l'autre de moins de 15 mm, de préférence de moins de 10 mm et de manière particulièrement préférée de moins de 5 mm, lorsque l'élément arqué (40) est fixé sur la partie de réception (20) au moyen de l'élément de fixation (48) et que le deuxième élément de travail (30) est inséré complètement dans la partie de guidage (74).
